(19) Europäisches Patentamt
European Patent Office
Office européen des brevets

(11) Veröffentlichungsnummer: 0 009 164
B1

(12) EUROPÄISCHE PATENTSCHRIFT

(45) Veröffentlichungstag der Patentschrift: 15.12.82

(51) Int. Cl.³: G 01 N 33/72

(21) Anmeldenummer: 79103262.6

(22) Anmeldetag: 04.09.79

# (54) Diagnostisches Mittel und dessen Verwendung zum Nachweis von Urobilinogen.

(30) Priorität: 14.09.78 DE 2839931

(43) Veröffentlichungstag der Anmeldung: 02.04.80 Patentblatt 80/7

(45) Bekanntmachung des Hinweises auf die Patenterteilung: 15.12.82 Patentblatt 82/50.

(84) Benannte Vertragsstaaten: AT BE CH DE FR GB IT LU NL SE

(56) Entgegenhaltungen:
DE - A - 2 130 559
DE - B - 2 251 402
FR - A - 2 190 278

(73) Patentinhaber: BEHRINGWERKE AKTIENGESELLSCHAFT
Postfach 1140
D-3550 Marburg/Lahn (DE)

(72) Erfinder: Hirsch, Wolfgang, Dr.
Pirolweg 12
D-3050 Wunstorf (DE)

(74) Vertreter: Meyer-Dulheuer, Karl-Hermann, Dr. et al,
HOECHST Aktiengesellschaft Zentrale
Patentabteilung Postfach 80 03 20
D-6230 Frankfurt/Main 80 (DE)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 009 164 B1

# Diagnostisches Mittel und dessen Verwendung zum Nachweis von Urobilinogen

Gegenstand der vorliegenden Erfindung ist ein diagnostisches Mittel zum Nachweis von Urobilinogen in Flüssigkeiten, vorzugsweise in biologischen Flüssigkeiten, insbesondere in Urin.

Es ist bereits bekannt, Urobilinogen mit einer Lösung von Dimethylaminobenzaldehyd in Salzsäure nachzuweisen. Dieser Nachweis, als Ehrlich-Reaktion bekannt, hat im Laufe der Zeit, obwohl er nicht sehr spezifisch ist, in der medizinischen Diagnostik erheblich an Bedeutung gewonnen. Der Nachweis von Urobilinogen im Urin gilt mittlerweile als Standardmethode für die Diagnose von Leber- und Gallenerkrankungen.

Im Rahmen der verbreiteten Anwendung von Schnelldiagnostika sind seit einiger Zeit auch Testpapiere zum Nachweis von Urobilinogen auf der Basis der Ehrlich-Reaktion entwickelt worden. Diese Papiere besitzen naturgemäß die Unspezifität der Ehrlich-Probe und haben zudem noch den Nachteil, daß die Farbreaktion sich sehr langsam entwickelt.

Bereits 1907 (Dissertation Karl Thomas, Freiburg) wurde beschrieben, daß Urobilinogen auch mit diazotierten Aminen reagiert. Diese sogenannte gelbe Diazoreaktion hat aber in der Folgezeit keinen Eingang in das ärztliche Labor gefunden. In neuerer Zeit (DE—C—21 30 559, DE—A—22 29 611, DE—A—23 64 844) sind jedoch Testpapiere zum Nachweis von Urobilinogen in Flüssigkeiten beschrieben worden, die auf der Diazoreaktion beruhen. Als Reagenzien werden aromatisch substituierte bzw. anellierte stabile Phenyl-, Pyrrol- und Pyrazoldiazoniumsalze beschrieben (FR—A—2 190 278, entsprechend DE—A—22 29 611), wobei die Summe der Hammett'schen Sigmawerte + 0,6 nicht übersteigt, oder auch substituierte Benzidinderivate verwendet DE—A—23 64 844). In einem anderen Patent (DE—C—21 30 559) wird die Verwendung stabiler Benzol-Diazoniumsalze beschrieben, die in ortho- oder para-Stellung mindestens eine mehratomige mesomeriefähige Elektronendonatorgruppe enthalten, wobei die Summe der Hammett'schen Sigmawerte aller Substituenten den Wert + 0,4 nicht überschreiten darf. Darüberhinaus wurde auch gefunden, daß eine aromatische Diazoniumverbindung mit einer Summe der Sigmawerte der Substituenten von + 0,77 ein brauchbares Reagenz zum Nachweis von Urobilinogen ergibt (DE—B—25 21 402). Bei der Nacharbeitung hat sich aber ergeben, daß die dort angegebene Verbindung offensichtlich nich stabil ist und in der fertigen Reagenzmischung im wesentlichen in einer substituierten Form vorliegt. In die Nachweisreaktion geht auch hier eine Verbindung ein, bei der die Summe der Hammett'schen Sigmawerte kleiner als + 0,4 ist.

Die auf der Diazoreaktion beruhenden Testpapiere sind im allgemeinen weniger störungsanfällig als die auf der Ehrlich-Reaktion beruhenden Papiere. Die bisher bekannten zeigen aber häufig auch noch mit Bilirubin, einem ebenfalls bei Gallen- und Lebererkrankungen im Urin auftretenden Gallenfarbstoff, eine Reaktion.

Überraschenderweise wurde nunmehr gefunden, daß es aromatische Diazoniumsalze mit einer Summe der Hammett'schen Sigmawerte der Substituenten von 0,62 oder größer gibt, die stabil sind und sich hervorragend zum Nachweis von Urobilinogen eignen. Insbesondere wurden hier die Verbindungen der allgemeinen Formel I gefunden, die spezifische und empfindliche Nachweismittel für Urobilinogen darstellen und einen verläßlichen Urobilinogennachweis auch bei Gegenwart von Bilirubin liefern.

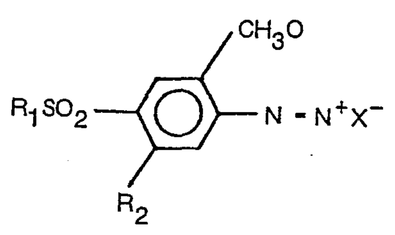

(I)

In der Formel I bedeutet

$R_1$ einen $CH_3$—, $C_2H_5$—, $CH_2$=CH—, $HOCH_2CH_2$— oder $CH_3OC_2H_4$-Rest

$R_2$ Wasserstoff oder einen $C_1$—$C_4$-Alkylrest und

$X^-$ ein stabilisierendes Anion.

$X^-$ kann bevorzugt ein Chlorid-, Sulfat-, Tetrafluoroborat-, Hexafluoroantimonat-, Hexafluoroantimonsulfonat-, Trifluormethylsulfonat-, Arylsulfonat- oder Carbonsäurerest sein.

Diazoniumsalze der Formel I sind ggf. in Verbindung mit einer oder mehreren festen Säuren und/oder einem Stabilisator und/oder einem Netzmittel und/oder einem optischen Aufheller besonders für die Verwendung auf einem saugfähigen Träger brauchbar.

Die erfindungsgemäßen Verbindungen reagieren mit Urobilinogen innerhalb weniger Sekunden zu roten Farbstoffen. Die Farbreaktion wird durch die natürlichen Harninhaltsstoffe, wie z.B. Harnindikan und Harnstoff nicht gestört. Auch mit Bilirubin reagieren die erfindungsgemäßen Diazoniumverbindungen nicht, so daß bei gleichzeitigem Vorhandensein von Bilirubin und Urobilinogen das letztere spezifisch nachgewiesen werden kann.

Das ist um so erstaunlicher, als in der DE—B—21 30 559, Spalte 4, Zeilen 26—29 der Hinweis gegeben wird, daß zur Unterdrückung der Reaktion mit Bilirubin als Indikator ein Diazoniumsalz relativ niedriger Elektrophilie zu verwenden ist. Demgegenüber wird hier mit Diazoniumverbindungen relativ hoher Elektrophilie eine große Spezifität bei gleichzeitiger Anwesenheit beider Inhaltsstoffe erreicht. Die Hammet'schen Sigmawerte der Verbindungen der Formel I betragen bei $R_2$ = Wasserstoff + 0,72 und bei $R_2 = C_1$—$C_4$-Alkyl-Rest + 0,65 bis + 0,62. Für die $CH_2$=CH—$SO_2$-Gruppe in 4-Stellung gibt es keine Literaturwerte; es ist aber ein zusätzlicher elektronenziehender Effekt gegenüber der $\beta$-Hydroxyäthylsulfonyl-Gruppe zu erwarten.

Vorzugsweise werden die Verbindungen der Formel I zur Herstellung von Testpapieren verwendet. Sie werden zu diesem Zweck mit einer Säure und ggf. mit Zusatzstoffen, wie Stabilisatoren, Netzmittel und Aufhellern auf einen saugfähigen Träger aufgebracht.

Zur Herstellung wird ein saugfähiger Träger, vorzugsweise Papier oder Polyestervlies, mit einer Lösung der o.g. Reagenzien in einem Gemisch aus einem organischen mit Wasser mischbaren Lösungsmittel und Wasser getränkt und anschließend in bewegter Luft zwischen 0 und 80°C getrocknet.

Die Diazoniumverbindungen der Formel I, die in Mengen von 0,02 bis 2 g, vorzugsweise, 0,1 bis 0,5 g/100 ml der Tränklösung zugesetzt werden, können nach den üblichen Verfahren der Diazochemie hergestellt werden oder werden in der Tränklösung aus dem entsprechenden aromatischen Amin (4-Methylsulfonyl-2-methoxy-anilin nach DE—A—21 14 578 und 2-Methoxy-5-methyl-4-($\beta$-hydroxyäthyl)-sulfonyl-anilin nach DE—B—11 50 163, Beispiel 1) nach in der Diazochemie bekannten Verfahren erzeugt.

Als feste Säuren, die in Mengen von 1—30 g, vorzugsweise 5—15 g/100 ml der Tränklösung zugesetzt werden, kommen organische aromatische und aliphatische Carbon- bzw. Sulfonsäuren allein oder in Mischung, auch mit anorganischen Säuren, in Betracht. Stabilisatoren, wie Naphthalin-(1,5)-disulfonsäure-Dinatriumsalz bzw. Natriumlaurylsulfat, sind aus der Diazochemie hinlänglich bekannt. Sie können in Mengen von 1—10 g, vorzugsweise 1—7 g/100 ml der Tränklösung zugesetzt werden.

Netzmittel wie Dodecylbenzosulfonsäure oder Natriumlaurylsulfat können in Mengen von 0,1—5 g, vorzugsweise 0,5—1 g/100 ml der Tränklösung zugesetzt werden.

Optische Aufheller, die eine Verbesserung der Ablesbarkeit ergeben, können in Mengen von 0,01—5 g, vorzugsweise 0,1—2 g/100 ml der Tränklösung zugegeben werden. In Betracht kommen Stilben-Derivate, wie sie unter der Bezeichnung Blankophor (R) von Bayer AG in den Handel gebracht werden.

Als Lösungsmittel kommt Wasser in Verbindung mit einem wassermischbaren organischen Lösungsmittel, vorzugsweise einem niederen Alkohol, z.B. Methanol, in Betracht, wobei das Verhältnis Wasser zu Lösungsmittel nicht kritisch ist, sondern lediglich von der Löslichkeit der Komponenten bestimmt wird.

Als saugfähige Träger können Filterpapiere, aber auch Vliese aus Polyamid bzw. Polyester oder anderen säurebeständigen Kunststoffen in Frage kommen. Das Material des saugfähigen Trägers ist aber nicht kritisch. Auch andere Materialisen, die in der Lage sind, die Tränklösung aufzunehmen, können verwendet werden. Die einzelnen Bestandteile der Rezeptur können selbstverständlich auch nacheinander auf den Träger aufgebracht werden, wenn die Loslichkeit oder besondere Umstände es erfordern sollten.

Die Verbindungen der Formel I können auch für einen Nachweis in Lösung verwendet werden. Man stellt sich dann nach der o.g. Vorschrift eine Lösung her, in die zweckmäßigerweise die Untersuchungslösung eingetropft wird. In diesem Fall ist die Verwendung eines optischen Aufhellers nicht nötig. Die Ablesung wird entweder im Vergleich mit einer Farbtafel oder mit einem Spektralphotometer durchgeführt.

Zur Durchführung des erfindungsgemäßen Verfahrens werden die nach der oben beschriebenen Methode hergestellten diagnostischen Mittel kurz in die zu untersuchende Flüssigkeit eingetaucht. Nach wenigen Sekunden kann man den Farbumschlag ggf. durch Vergleich mit einer Farbskala ablesen.

In den folgenden Beispielen wird das erfindungsgemäße diagnostische Mittel näher erläutert:

Filterpapier Schleicher & Schüll 2316 wird mit je einer der in den Beispielen 1 und 2 angegebenen Lösungen getränkt und bei Raumtemperatur getrocknet.

Beispiel 1

| | |
|---|---|
| 4-Methylsulfonyl-2-methoxybenzol-diazoniumtetrafluoroborat | 0,1 g |
| Methanol | 10 ml |
| meta-Phosphorsäure | 10 g |
| Zitronensäure-1-hydrat | 3 g |
| Dodecylbenzolsulfonsäure | 1 g |
| Wasser | ca. 100 ml |

Das nach diesem Beispiel hergestellte Testpapier zeigt 0,5 mg Urobilinogen/100 ml Lösung an und gibt mit urobilinogenhaltigem Urin eine rote Färbung.

In gleicher Weise lassen sich Testpapiere herstellen unter Verwendung von je 0,1 g der folgenden Diazoniumverbindungen:

4-Äthylsulfonyl-2-methoxybenzoldiazoniumtrifluormethylsulfonat
4-Vinylsulfonyl-2-methoxybenzoldiazoniumtetrafluoroborat
4-(β-Hydroxyäthyl)sulfonyl-2-methoxybenzodiazoniumtetrafluoroborat
4-(Methoxyäthyl)sulfonyl)-2-methoxybenzoldiazoniumarylsulfonat
4-Methylsulfonyl-2-methoxy-5-methyl-benzoldiazoniumtetrafluoroborat
4-Äthylsulfonyl-2-methoxy-5-methyl-benzoldiazoniumtetrafluoroborat
4-Vinylsulfonyl-2-methoxy-5-methyl-benzoldiazoniumtetrafluoroborat
4-(β-Hydroxyäthyl)sulfonyl-2-methoxy-5-methyl-benzoldiazoniumtetrafluoroborat
4-(Methoxyäthyl)sulfonyl-2-methoxy-5-methyl-benzoldiazoniumtetrafluoroborat.

Die erhaltenen Testpapiere geben gleichfalls mit urobilinogenhaltigem Urin eine rote Färbung.

Beispiel 2

10 g meta Phosphorsäure
3 g Zitronensäure-1-hydrat
1 g Naphthalin-(1,5)-disulfonsäure-Dinatriumsalz
1 g Dodecylbenzolsulfonsäure
1 g optischer Aufheller

werden in
90 ml destilliertem Wasser gelöst, auf 4—5°C gekühlt und mit einer Aufschlämmung von
0,15 g 4-(β-Hydroxyäthyl)sulfonyl-2-methoxy-5-methyl-anilin in
10 ml Methanol
zusammengegeben. Anschließend werden
ca. 2 ml Isoaymylnitril zugefügt.

In dieser Lösung wird das Filtrierpapier getränkt und kann nach dem Trocknen zum Nachweis von Urobilinogen verwendet werden.

Ein nach diesem Beispiel hergestelltes Testpapier zeigt nach Eintauchen in normalen Urin abhängig von seinem Urobilinogen-Gehalt eine schwach rosa Färbung. Mit stark urobilinogen-haltigem Urin ergeben sich rote bis tiefrote Färbungen.

Die gleichen Färbungen — allerdings durch das vorhandene Bilirubin etwas gelblicher — werden bei Papieren nach Beispiel 1 und 2 erhalten, wenn den Urinproben vor dem Test jeweils 10 mg Bilirubin/100 ml Lösung zugesetzt werden. Dagegen zeigen die Testmittel mit urobilinogenfreiem aber bilirubinhaltigem Urin innerhalb vergleichbarer Zeiten keine Farbreaktion. Während noch nach 5 Min. keine Färbung zu beobachten ist, kann nach 10 Min. eine Grünfärbung auftreten.

In gleicher Weise lassen sich Testpapiere herstellen unter Verwendung von je 0,15 g der folgenden Anilin-Verbindungen:

4-Methylsulfonyl-2-methoxy-anilin
4-Äthylsulfonyl-2-methoxy-anilin
4-Vinylsulfonyl-2-methoxy-anilin
4-(β-Hydroxyäthyl)sulfonyl-2-methoxy-anilin
4-(Methoxyäthyl)sulfonyl-2-methoxy-anilin
4-Methylsulfonyl-2-methoxy-5-methyl-anilin
4-Äthylsulfonyl-2-methoxy-5-methyl-anilin
4-Vinylsulfonyl-2-methoxy-5-methyl-anilin

4-(Methoxyäthyl)sulfonyl-2-methoxy-5-methyl-anilin

Die erhaltenen Testpapiere geben gleichfalls mit urobilinogenhaltigem Urin eine rote Färbung und zeigen mit urobilinogenfreiem aber bilirubinhaltigem Urin keine Farbreaktion.

**Patentansprüche für die Vertragsstaaten: BE CH DE FR GB IT LU NL SE**

1. Verwendung aromatischer Diazoniumsalze, bei denen die Summe der Hammett'schen Sigmawerte aller Substituenten, 0,62 oder größer ist, für den Nachweis von Urobilinogen in Flüssigkeiten, vorzugsweise in biologischen Flüssigkeiten, insbesondere in Urin.

2. Verwendung einer Verbindung der Formel I

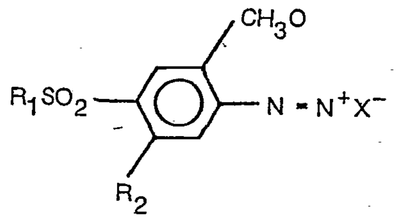

gemäß Anspruch 1

in welcher

$R_1$ einen $CH_3$—, $C_2H_5$—, $CH_2$=CH—, $HOC_2H_4$— oder $CH_3OC_2H_4$-Rest
$R_2$ Wasserstoff oder einen $C_1$—$C_4$-Alkyl-Rest und
$X^-$ ein stabilisierendes Anion bedeutet, zum Nachweis von Urobilinogen.

3. Verwendung von Verbindungen gemäß Anspruch 2, in welcher $X^-$ einen Chlorid-, Sulfat-, Tetrafluoroborat-, Hexafluoroantimonat-, Hexafluoroantimonsulfonat, Trifluormethylsulfonat-, Arylsulfonat- oder Carbonsäure-rest bedeutet, für den Nachweis von Urobilinogen.

4. Diagnostisches Mittel zum Nachweis von Urobilinogen dadurch gekennzeichnet, daß es ein Diazoniumsalz gemäß Anspruch 1—3, eine oder mehrere feste Säuren und ggf. ein Netzmittel sowie einen oder mehrere Stabilisatoren enthält.

5. Mittel gemäß Anspruch 4, dadurch gekennzeichnet, daß es als Diazoniumsalz eine Verbindung der allgemeinen Formel I, als Säure ein Gemisch aus meta-Phosphorsäure und Zitronensäure, als Netzmittel Dodecylbenzolsulfonsäure und als Stabilisator Naphthalin-(1,5)-Disulfonsäure-Dinatriumsalz, sowie ggf. einen optischen Aufheller enthält.

6. Mittel gemäß einem der Ansprüche 4 oder 5, dadurch gekennzeichnet, daß es auf einem saugfähigen Träger imprägniert ist.

**Patentansprüche für den Vertragsstaat: AT**

1. Verfahren für den Nachweis von Urobilinogen in Flüssigkeiten, vorzugsweise in biologischen Flüssigkeiten, insbesondere in Urin, dadurch gekennzeichnet, daß die Probe mit einem aromatischen Diazoniumsalz, bei dem die Summe der Hammett'schen Sigmawerte aller Substituenten 0,62 oder größer ist, in Berührung gebracht wird.

2. Verfahren nach Anspruch 1, d. h., daß das aromatische Diazoniumsalz eine Verbindung der Formel I

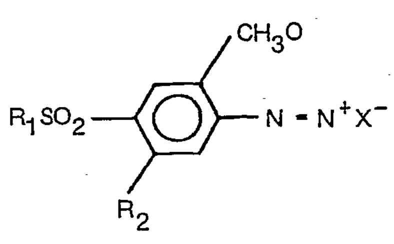

ist, in welcher

$R_1$ einen $CH_3$—, $C_2H_5$—, $CH_2$=CH—, $HOC_2H_4$ oder $CH_3OC_2H_4$-Rest
$R_2$ Wasserstoff oder einen $C_1$—$C_4$-Alkyl-Rest und
$X^-$ ein stabilisierendes Anion bedeutet.

3. Verfahren nach Anspruch 1, dadurch gekennzeichnet, daß das aromatische Diazoniumsalz eine Verbindung der Formel I ist, in welcher $X^-$ einen Chlorid-, Sulfat-, Tetrafluoroborat-, Hexafluoroantimonat-, Hexafluoroantimonsulfonat-, Trifluormethylsulfonat-, Arylsulfonat- oder Carbonsäurerest bedeutet.

4. Diagnostische Mittel zum Nachweis von Urobilinogen dadurch gekennzeichnet, daß es ein Diazoniumsalz gemäß Anspruch 1—3, eine oder mehrere feste Säuren ggf. ein Netzmittel sowei einen oder mehrere Stabilisatoren enthält.

5. Mittel gemäß Anspruch 4, dadurch gekennzeichnet, daß es als Diazoniumsalz eine Verbindung der allgemeinen Formel I, als Säure ein Gemisch aus meta-Phosphorsäure und Zitronensäure, als Netzmittel Dodecylbenzolsulfonsäure und als Stabilisator Naphthalin-(1,5)-Disulfonsäure-Dinatriumsalz, sowie ggf. einen optischen Aufheller enthält.

6. Mittel gemäß einem der Ansprüche 4 oder 5, dadurch gekennzeichnet, daß es auf einem saugfähigen Träger imprägniert ist.

**Revendications pour les Etats contractants: BE CH DE FR GB IT LU NL SE**

1. Utilisation de sels de diazonium aromatiques, dans lesquels la somme des valeurs sigma de Hammett de tous les substituants est de 0,62 ou plus, pour la détection de l'urobilinogène dans des liquides, avantageusement dans des liquides biologiques, en particulier dans l'urine.

2. Utilisation d'un composé de formule I

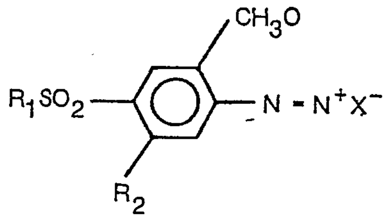

selon la revendication 1, dans laquelle:

$R_1$ représente un radical $CH_3$, $C_2H_5$, $CH_2{=}CH$, $HOC_2H_4—$ ou $CH_3OC_2H_4$,
$R_2$ représente l'hydrogène ou un radical alkyle en $C_1—C_4$ et
$X^-$ représente un anion stabilisant,

pour la détection de l'urobilinogène.

3. Utilisation de composés selon la revendication 2 dans lesquels $X^-$ représente un groupe chlorure, sulfate, tétrafluoroborate, hexafluoroantimoniate, hexafluoroantimonosulfonate, trifluorométhylsulfonate, arylsulfonate ou acide carboxylique, pour la détection de l'urobilinogène.

4. Réactif pour diagnostic pour la détection de l'urobilinogène, caractérisé en ce qu'il contient un sel de diazonium selon l'une quelconque des revendications 1 à 3, un ou plusieurs acides solides et éventuellement un agent mouillant ainsi qu'un ou plusieurs stabilisants.

5. Réactif selon la revendication 4, caractérisé en ce qu'il contient comme sel de diazonium un composé de formule générale I, comme acide un mélange d'acide métaphosphorique et d'acide citrique, comme agent mouillant l'acide dodécylbenzène sulfonique et comme stabilisant le sel disodique de l'acide naphtalène-(1,5)-disulfonique, ainsi qu'éventuellement un azurant optique.

6. Réactif selon l'une des revendications 4 et 5, caractérisé en ce qu'on en imprègne un support absorbant.

**Revendications pour l'Etat contractant: AT**

1. Procédé de détection de l'urobilinogène dans les liquides, avantageusement dans des liquides biologiques, en particulier dans l'urine, caractérisé en ce que l'échantillon est amené en contact avec un sel de diazonium aromatique pour lequel la somme des valeurs sigma de Hammett de tous les substituants est de 0,62 ou plus.

2. Procédé selon la revendication 1, caractérisé en ce que le sel de diazonium aromatique est un composé de formule I

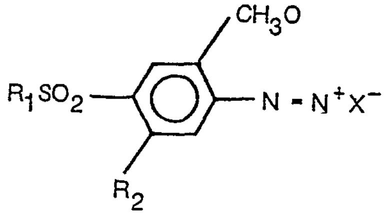

dans laquelle

$R_1$ représente un radical $CH_3$, $C_2H_5$, $CH_2{=}CH$, $HOC_2H_4$— ou $CH_3OC_2H_4$,

$R_2$ représente un hydrogène ou un radical alkyle en $C_1$—$C_4$, et

$X^-$ représente un anion stabilisant.

3. Procédé selon la revendication 1, caractérisé en ce que le sel de diazonium aromatique est un composé de formule 1 dans lequel $X^-$ représente un groupe chlorure, sulfate, tétrafluoroborate, hexafluoroantimoniate, hexafluoroantimonosulfonate, trifluorométhylsulfonate, arylsulfonate ou acide carboxylique.

4. Réactif pour diagnostic pour la détection de l'urobilinogène, caractérisé en ce qu'il contient un sel de diazonium selon l'une quelconque des revendications 1 à 3, un ou plusieurs acides solides et éventuellement un agent mouillant ainsi qu'un ou plusieurs stabilisants.

5. Réactif selon la revendication 4, caractérisé en ce qu'il contient comme sel de diazonium un composé de formule générale I, comme acide un mélange d'acide métaphosphorique et d'acide citrique, comme agent mouillant l'acide dodécylbenzène sulfonique et comme stabilisant le sel disodique le l'acide naphtalène-(1,5)-disulfonique, ainsi qu'éventuellement un azurant optique.

6. Réactif selon l'une des revendications 4 ou 5, caractérisé en ce qu'on en imprègne un support absorbant.

**Claims for the Contracting States: BE CH DE FR GB IT LU NL SE**

1. The use of aromatic diazonium salts in which the sum of the Hammet sigma-values of all substituents is 0.62 or greater for the proof of urobilinogen in liquids, preferably in biological fluids, in particular in urine.

2. The use of a compound of the formula I

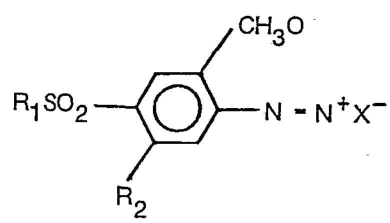

according to claim 1

in which

$R_1$ represents a $CH_3$—, $C_2H_5$—, $CH_2{=}CH$—, $HOC_2H_4$—, or $CH_3OC_2H_4$-radical,

$R_2$ represents hydrogen or a $C_1$—$C_4$ radical and

$X^-$ represents a stabilizing anion, for the proof of urobilinogen.

3. The use of compounds as claimed in claim 2, in which $X^-$ represents a chloride, sulfate, tetrafluoroborate, hexafluoroantimonate, hexafluoroantimony sulfonate, trifluoromethylsulfonate, arylsulfonate or carboxylic acid radical, for the proof of urobilinogen.

4. Diagnostic agent for the proof of urobilinogen which contains a diazonium salt as defined in claims 1 to 3, one or several solid acids and optionally a wetting agent and one or several stabilizers.

5. A diagnostic agent as claimed in claim 4, which contains a compound of the general formula I as the diazonium salt, a mixture of meta-phosphoric acid and citric acid as the acid, dodecyl-benzenesulfonic acid as the wetting agent and naphthalene-(1,5)-disulfonic acid disodium salt as the stabilizer, and optionally an optical brightener.

6. An agent as claimed in any one of claims 4 or 5 which is impregnated onto an adsorbing carrier.

**Claims for the Contracting State: AT**

1. Process for determining Urobilinogen in liquids, particularly in biological liquids, especially in urine, which comprises contacting the sample with an aromatic diazonium salt in which the sum of the Hammet sigma-values of all substituents is 0.62 or greater.

2. Process according to claim 1 in which the aromatic diazonium salt is a compound of the formula I

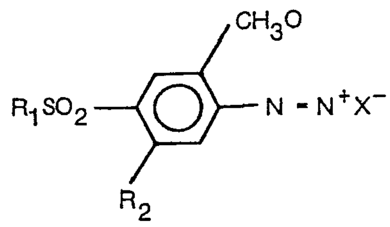

(I)

in which

$R_1$ represents a $CH_3$—, $C_2H_5$—, $CH_2$=CH—, $HOC_2H_4$—, or $CH_3OC_2H_4$-radical,
$R_2$ represents hydrogen or a $C_1$—$C_4$ radical and
$X^-$ represents a stabilizing anion.

3. Process according to claim 1 in which the aromatic diazonium salt is a compound of the formula I, in which $X^-$ represents a chloride, sulfate, tetrafluoroborate, hexafluoroantimonate, hexafluoroantimony sulfonate, trifluoromethylsulfonate, arylsulfonate or carboxylic acid radical.

4. Diagnostic agent for the proof of urobilinogen which contains a diazonium salt as defined in claims 1 to 3, one or several solid acids and optionally a wetting agent and one or several stabilizers.

5. An agent as claimed in claim 4, which contains a compound of the general formula I as the diazonium salt, a mixture of meta-phosphoric acid and citric acid as the acid, dodecyl-benzenesulfonic acid as the wetting agent and naphthalene-(1,5)-disulfonic acid disodium salt as the stabilizer, and optionally an optical brightener.

6. An agent as claimed in any one of claims 4 or 5 which is impregnated onto an adsorbing carrier.